# FASCICULE DE BREVET EUROPEEN

(11) **EP 2 364 692 B1**
(45) Date de publication et mention de la délivrance du brevet: **25.10.2017**
(21) Numéro de dépôt: 11167661.5
(22) Date de dépôt: 01.12.2008
(51) Int. Cl.: A61K 31/327, A61K 31/192, A61P 17/10, A61K 8/04, A61K 8/36, A61K 8/368, A61K 8/38, A61K 8/73, A61K 8/81, A61Q 5/00, A61Q 19/00, A61Q 19/08, A61K 9/06, A61K 9/00, A61K 47/32, A61K 47/10, A61K 47/14, A61K 47/36, A61K 47/38, A61K 47/44, A61K 47/24

(54) **Compositions comprenant au moins un derive de l'acide naphtoïque, du peroxyde de benzoyle et au moins un agent filmogene, leurs procedes de preparation, et leurs utilisations**
Zusammensetzungen, die mindestens ein Naphtoesäurederivat, Benzoylperoxid und mindestens eine filmbildende Substanz enthalten, ihre Herstellungsverfahren und ihre Anwendungen
Compositions including at least one derivative of naphtoic acid, benzoyl peroxide and at least one film-forming agent, methods for preparing same and uses thereof

(30) Priorité: 30.11.2007 US 4763 P
(43) Date de publication de la demande: 14.09.2011
(62) Demande divisionnaire de: 08863372.2
(73) Titulaire: Galderma Research & Development, 06410 Biot (FR)
(72) Inventeur: Mallard, Claire, 06250 Mougins (FR)
(74) Mandataire: Casalonga

(56) Documents cités:
- EP-A1- 1 967 180
- EP-A2- 0 386 960
- WO-A1-2008/006848
- WO-A1-2008/006888
- WO-A1-2008/065306
- WO-A2-2007/002831
- WO-A2-2007/031883
- WO-A2-2008/017914
- WO-A2-2008/087354
- US-A- 6 159 493
- US-A1- 2003 170 196
- KORKUT C ET AL: "BENZOYL PEROXIDE, ADAPALENE, AND THEIR COMBINATION IN THE TREATMENT OF ACNE VULGARIS", JOURNAL OF DERMATOLOGY, JAPANESE DERMATOLOGICAL ASSOCIATION, TOKYO, JP, vol. 32, no. 3, 1 janvier 2005 (2005-01-01), pages 169-173, XP009075640, ISSN: 0385-2407
- ANONYMOUS: "GUIDE VIDAL DES MEDICAMENTS", 1 janvier 1997 (1997-01-01), EDITIONS DU VIDAL, FR, PAGE 481, XP002545403, * *
- NAIR BINDU: "Final report on the safety assessment of polyvinyl alcohol", INTERNATIONAL JOURNAL OF TOXICOLOGY, vol. 17, no. SUPPL. 5, 1998, pages 67-92, XP008165175, ISSN: 1091-5818

## Description

La présente invention se rapporte à des compositions pour application topique, des procédés de préparation de telles compositions, et leurs utilisations en tant que produits cosmétiques ou pharmaceutiques, lesdites compositions étant destinées, en particulier, au traitement de l'acné.

L'acné est une pathologie multifactorielle fréquente qui atteint la peau riche en glandes sébacées (visage, région scapulaire, bras et régions intertrigineuses). Elle est la plus fréquente des dermatoses. Les cinq facteurs pathogéniques suivants jouent un rôle déterminant dans la constitution de l'acné :
1. la prédisposition génétique;
2. la surproduction de sébum (séborrhée);
3. les androgènes;
4. les troubles de la kératinisation folliculaire (comédogenèse);
5. la colonisation bactérienne et les facteurs inflammatoires.

Il existe plusieurs formes d'acné, ayant toutes en commun l'atteinte des follicules pilosébacés. On peut citer notamment, l'acné conglobata, l'acné chéloïde de la nuque, l'acné médicamenteuse, l'acné miliaire récidivante, l'acné nécrotique, l'acné neonatorum, l'acné prémenstruelle, l'acné professionnelle, l'acné rosacée, l'acné sénile, l'acné solaire, et l'acné vulgaire.

L'acné vulgaire, appelée également acné juvénile polymorphe, est la plus courante. Elle comprend quatre stades, mais le passage par tous les stades n'est pas obligatoire :
- Le stade 1 correspond à l'acné comédonienne caractérisée par un grand nombre de comédons ouverts et/ou fermés, et de microkystes.
- Le stade 2, ou acné papulopustuleuse, est de gravité légère à modérée. Elle est caractérisée par la présence de comédons ouverts et/ou fermés, de microkystes, mais également de papules rouges et de pustules. Elle touche principalement le visage et laisse peu de cicatrices.
- Le stade 3, ou acné papulocomédonienne, est plus grave et s'étend au dos, au thorax et aux épaules. Elle est accompagnée d'un plus grand nombre de cicatrices.
- Le stade 4, ou acné nodulokystique, s'accompagne de nombreuses cicatrices. Elle présente des nodules ainsi que des pustules volumineuses violacées et douloureuses.

Les différentes formes d'acné décrites précédemment peuvent être traitées par des actifs tels que les anti-séborrhéiques et les anti-infectieux, par exemple le peroxyde de benzoyle (notamment le produit Eclaran® commercialisé par la société Pierre Fabre), par des rétinoïdes tels que la trétinoïne (notamment le produit Retacnyl® commercialisé par la société Galderma) ou l'isotrétinoïne (produit Roaccutane® commercialisé par les Laboratoires Roche), ou par des dérivés d'acide naphtoïque. Les dérivés d'acide naphtoïque tels que notamment l'acide 6-[3-(11-adamantyl)-4-méthoxyphényl]-2-naphtoïque), communément appelé adapalène (produit Differine® commercialisé par la société Galderma), sont largement décrits et reconnus comme des principes actifs aussi efficaces que la trétinoïne pour le traitement de l'acné.

La combinaison de plusieurs traitements locaux (antibiotiques, rétinoides, peroxydes, zinc) est également utilisée en dermatologie pour permettre d'augmenter l'efficacité des principes actifs et de diminuer leur toxicité (Cunliffe W.J., J. Dermatol. Treat., 2000, 11 (suppl2), S13-S14) mais l'application multiple de différents produits dermatologiques peut être assez lourde et astreignante pour le patient.

On comprend donc l'intérêt de chercher à obtenir un nouveau traitement efficace sur les affections dermatologiques dans une composition stable offrant une bonne cosméticité, permettant une application unique et une utilisation agréable pour le patient.

Parmi cette panoplie de thérapeutiques proposée à l'homme du métier, rien ne l'encourageait à associer, dans la même composition, le peroxyde de benzoyle et un rétinoide.

En effet, la formulation d'une telle composition pose plusieurs problèmes.

Tout d'abord, l'efficacité du peroxyde de benzoyle est liée à sa décomposition lorsqu'il est mis en contact avec la peau. En effet, ce sont les propriétés oxydantes des radicaux libres produits lors de cette décomposition qui conduisent à l'effet désiré. Aussi, afin de maintenir au peroxyde de benzoyle une efficacité optimale, il est important de prévenir sa décomposition avant utilisation, c'est à dire durant le stockage.

Or le peroxyde de benzoyle est un composé chimique instable qui rend difficile sa formulation dans des produits finis.

La solubilité et la stabilité du peroxyde de benzoyle ont été étudiées par Chellquist *et al.* dans l'éthanol, le propylène glycol et différents mélanges de polyéthylène glycol 400 (PEG 400) et d'eau (Chellquist E.M. et Gorman W.G., Pharm. Res., 1992, Vol 9: 1341-1346).

Ce document précise par ailleurs que la stabilité du peroxyde de benzoyle est fortement influencée par la composition chimique de la formulation et par la température de stockage. Le peroxyde de benzoyle est extrêmement réactif et se dégrade en solution à basse température en raison de l'instabilité de sa liaison peroxyde.

Les auteurs constatent ainsi que le peroxyde de benzoyle en solution se dégrade plus ou moins rapidement dans tous les solvants étudiés en fonction du type de solvant et de sa concentration.

Les temps de dégradation du peroxyde de benzoyle dans le PEG 400 (0.5 mg/g), dans l'éthanol et dans le propylène glycol sont respectivement de 1,4, 29 et 53 jours à 40°C.

Une telle dégradation ne permet pas la préparation d'un produit destiné à la vente.

Une autre difficulté à surmonter pour la préparation d'une composition comprenant à la fois du peroxyde de benzoyle et un rétinoïde est que la plupart des rétinoïdes sont particulièrement sensibles à l'oxydation naturelle, à la lumière visible et aux ultra-violets, et le peroxyde de benzoyle étant un oxydant fort, la compatibilité chimique de ces composés dans une même formulation pose de nombreux problèmes de stabilité du point de vue physique et chimique.

Une étude de stabilité de deux rétinoïdes a été réalisée en combinant deux produits commercialisés, l'un contenant un rétinoïde (trétinoine ou adapalène) et le second à base de peroxyde de benzoyle *(*B. Martin et al., Br.J.Dermatol. (1998) 139, (suppl.52), 8-11*).*

La présence de la formulation à base de peroxyde de benzoyle provoque une dégradation très rapide des rétinoides sensibles à l'oxydation : on mesure que 50% de la trétinoine se dégrade en 2 heures, et 95% en 24 heures. Dans la composition dans laquelle le rétinoïde est l'adapalène, aucune dégradation de l'adapalène n'a été mesurée pendant 24 heures. Cette étude confirme que le peroxyde de benzoyle se dégrade et dégrade les rétinoïdes sensibles à l'oxydation au cours du temps en relarguant progressivement de l'acide benzoïque dans des produits finis.

Or il est clair que la dégradation du peroxyde de benzoyle et des rétinoïdes n'est pas souhaitable dans la mesure où elle nuit à l'efficacité de la composition les contenant.

Rien n'incitait à associer ces deux agents actifs afin d'obtenir une composition stable sachant qu'il était usuellement connu que la présence du peroxyde de benzoyle déstabilisait chimiquement et physiquement ce type de composition.

De plus, l'homme de l'art cherche en permanence à améliorer l'efficacité et la tolérance des compositions contenant le peroxyde de benzoyle et un dérivé de l'acide naphtoïque. Une des solutions pour améliorer l'efficacité est d'augmenter les quantités d'actifs présents dans la composition ou d'augmenter les durées de traitement. De telles modifications entraînent généralement une augmentation de l'irritation induite. C'est pourquoi il est nécessaire de réaliser des compositions pouvant encore améliorer la tolérance des principes actifs.

La demande US 2003/170196 décrit une composition comprenant, dans un milieu physiologiquement acceptable, au moins un rétinoïde, du peroxyde de benzoyle dispersé et au moins un gélifiant pH-indépendant, ainsi que ses utilisations thérapeutiques notamment pour traiter les affections dermatologiques.

La demande WO 2007/002831 décrit des compositions topiques et leur utilisation pour le traitement de divers troubles ou affections cutanées. Ces compositions comprennent un mélange stable au stockage d'une composition contenant du peroxyde de benzoyle avec un antibiotique ou un sel ou un ester de celui-ci, et un rétinoïde ou d'un sel pharmaceutiquement acceptable de celui-ci. Ces compositions se caractérisent en ce qu'elles ont un pH final allant de 3 à 8 et une viscosité inférieure à la viscosité de la composition contenant un peroxyde de benzoyle avant mélange.

Un problème que se propose de résoudre l'invention est de réaliser des compositions stables et moins irritantes que celles de l'art antérieur. De telles compositions doivent de plus favoriser la pénétration topique des principes actifs sous forme dispersée.

Il se trouve que la demanderesse a mis en évidence de manière surprenante que des ingrédients connus pour conférer à une composition un effet filmogène peuvent également améliorer la tolérance de la combinaison de deux principes actifs irritants, tels que les actifs anti-acnéiques et notamment le peroxyde de benzoyle et les dérivés de l'acide naphtoïque, tel que l'adapalène.

Ainsi, un des buts de la présente invention est de proposer une composition pour application topique particulièrement efficace, comprenant au moins un dérivé d'acide naphtoïque et du peroxyde de benzoyle, sans présenter d'effet manifestement irritant empêchant son utilisation à plus ou moins long terme par le sujet.

L'invention a pour premier objet une composition pour application topique comprenant, dans un milieu physiologiquement acceptable, au moins un dérivé de l'acide naphtoïque et du peroxyde de benzoyle et au moins un agent filmogène, ledit dérivé d'acide naphtoïque étant sous une forme dispersée dans ladite composition.

Ainsi, l'invention a pour premier objet une composition, de préférence pharmaceutique, notamment pour application topique, comprenant dans un milieu physiologiquement acceptable au moins :
(i) un dérivé de l'acide naphtoïque,
(ii) du peroxyde de benzoyle, et
(iii) un agent filmogène,
ledit dérivé d'acide naphtoïque et ledit peroxyde de benzoyle étant sous une forme dispersée dans ladite composition.

L'agent filmogène employé dans l'invention est choisi parmi les alcools polyvinyliques.

Par actif sous forme dispersée selon l'invention, on entend un principe actif sous forme de particules solides, mises en suspension dans un véhicule donné. De telles particules ont notamment une taille supérieure à 10 µm. De façon avantageuse, la granulométrie du rétinoïde et du peroxyde de benzoyle est telle qu'au moins 80% en nombre des particules, et de préférence au moins 90% en nombre des particules, ont un diamètre inférieur à 25 µm et au moins 99% en nombre des particules ont un diamètre inférieur à 100 µm.

Elle a pour deuxième objet un procédé de préparation d'une composition pour application topique, caractérisé en ce qu'il comprend l'étape de mélange d'un véhicule physiologiquement acceptable comprenant au moins un dérivé de l'acide naphtoïque et du peroxyde de benzoyle avec au moins un agent filmogène, ledit dérivé d'acide naphtoïque et le peroxyde de benzoyle étant sous une forme dispersée dans ladite composition. Par véhicule physiologiquement acceptable, on entend un véhicule compatible avec la peau, les muqueuses et/ou les phanères.

Enfin, elle a pour troisième objet l'utilisation d'une composition telle que décrite ci-dessus pour la préparation d'un médicament destiné au traitement et/ou à la prévention des affections dermatologiques liées à un désordre de la kératinisation portant sur la différenciation et sur la prolifération cellulaire, et notamment pour prévenir et/ou traiter les acnés comédoniennes, vulgaires, papulocomédoniennes, nodulokystiques, les acnés polymorphes, les acnés rosacées, les acnés, conglobata, les acnés séniles, ou encore les acnés secondaires telles que l'acné solaire, médicamenteuse ou professionnelle.

Lorsqu'une composition comprend, dans un milieu physiologiquement acceptable, au moins un dérivé de l'acide naphtoïque et du peroxyde de benzoyle et au moins un agent filmogène, ledit dérivé d'acide naphtoïque et le peroxyde de benzoyle étant sous une forme dispersée dans ladite composition, elle présente une très bonne tolérance sans modifier la quantité d'actif ayant pénétré dans la peau.

La composition selon l'invention comprend au moins un dérivé de l'acide naphtoïque, du péroxyde de benzoyle et au moins un agent filmogène choisi parmi les alcools polyvinyliques. L'acide naphtoïque est un composé de formule

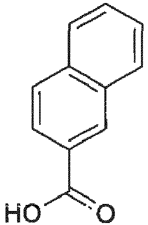

Par dérivé de l'acide naphtoïque, on entend les composés de formule (1): 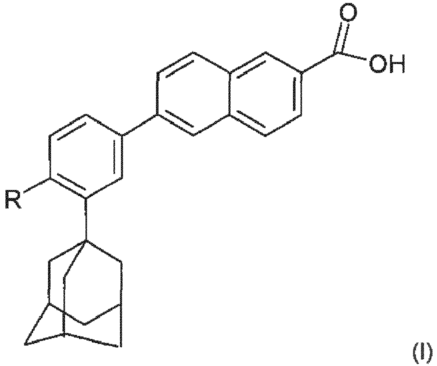 où R représente un atome d'hydrogène, un radical hydroxyle, un radical alkyle, ramifié ou non, ayant de 1 à 4 atomes de carbone, un radical alkoxy ayant de 1 à 10 atomes de carbone ou un radical cycloaliphatique substitué ou non.

Par radical alkyle linéaire ou ramifié ayant de 1 à 4 atomes de carbone, on entend de préférence les radicaux méthyle, éthyle, propyle et butyle.

Par radical alkoxy ayant de 1 à 10 atomes de carbone, on entend de préférence les radicaux méthoxy, éthoxy, propoxy, butoxy, hexyloxy et décyloxy.

Par radical cycloaliphatique, on entend de préférence les radicaux mono ou polycyclique tel que le radical methyl-1 cyclohexyle ou le radical 1-adamantyle.

Parmi les dérivés de l'acide naphtoïque susceptibles d'entrer dans les compositions selon l'invention, on choisira avantageusement l'acide 6-[3-(1-adamantyl)-4-methoxyphenyl]-2-naphtoïque (adapalène), l'acide 6-[3-(1-adamantyl)-4-hydroxyphenyl]-2-naphtoïque, l'acide 6-[3-(1-adamantyl)-4-decyloxyphenyl]-2-naphtoïque et l'acide 6-[3-(1-adamantyl)-4-hexyloxyphenyl]-2-naphtoïque.

Les dérivés de l'acide naphtoïque précités se présentent généralement sous une forme dispersée dans la composition selon l'invention. Les dérivés de l'acide naphtoïque insolubles sont ainsi répartis de façon homogène dans la composition selon l'invention.

Dans les compositions selon l'invention, les dérivés de l'acide naphtoïque sont utilisés à des concentrations inférieures ou égales à 10 % en poids par rapport au poids total de la composition, et de préférence sont comprises entre 0,001% et 10% en poids par rapport au poids total de la composition et, préférentiellement entre 0,01% et 5%, plus préférentiellement, entre 0,05% et 2%, et tout préférentiellement de 0,1 % à 0,3% en poids par rapport au poids total de la composition.

Dans l'ensemble du présent texte, à moins qu'il ne soit spécifié autrement, il est entendu que lorsque des intervalles de concentrations sont donnés, ils incluent les bornes supérieures et inférieures dudit intervalle.

De façon avantageuse, le dérivé de l'acide naphtoïque utilisé dans les compositions selon l'invention est l'acide 6-[3-(1-adamantyl)-4-methoxyphenyl]-2-naphtoïque (adapalène). De manière préférée, dans le cas de l'adapalène, la composition selon l'invention comprend entre 0.001% et 5% et avantageusement entre 0.01 et 1 % en poids d'adapalène par rapport au poids total de la composition, préférentiellement entre 0.01% et 0.5%, de préférence, entre 0.1% et 0.4 % en poids d'adapalène, encore plus préférentiellement à 0.1 % ou à 0.3 % en poids d'adapalène.

La composition comprend également du peroxyde de benzoyle (BPO).

Dans les compositions selon l'invention, le peroxyde de benzoyle est utilisé à des concentrations allant de 1% à 10 % en poids, plus particulièrement de 2% à 7% en poids, encore plus préférentiellement de 2,5% à 5 % en poids par rapport au poids total de la composition.

Le peroxyde de benzoyle pourra aussi bien être utilisé sous la forme libre ou bien sous une forme encapsulée sous forme adsorbée sur, ou absorbée dans, tout support poreux.
Il peut s'agir par exemple - de peroxyde de benzoyle encapsulé dans un système polymérique constitué de microsphères poreuses, comme par exemple des microéponges vendues sous le nom de Microsponges P009A Benzoyle peroxyde par la société Cardinal Health.

La composition selon l'invention comprend en outre au moins un alcool polyvinylique comme agent filmogène.

Par agent filmogène, on entend un polymère hydrophile ionique ou non-ionique de masse moléculaire au moins supérieure à 10000, qui, lors de l'application sur la peau, forme un film continu. La demanderesse a mis en évidence qu'un tel agent filmogène, confère une meilleure tolérance à la composition le contenant.

Comme exemple d'alcools polyvinyliques, on peut citer les alcools polyvinyliques présentant un degré de polymérisation compris entre 500 et 5000, un degré d'hydrolyse compris entre 85 et 89%, une viscosité comprise entre 20 et 65 mPa.s (4% (w/w) dans l'eau à 20°C). Plus précisément, on peut citer à titre d'exemple, le Mowiol 40-88 vendu par la société Sigma Aldrich présentant un degré de polymérisation de 4200, un degré d'hydrolyse compris entre 86.7 et 88.7% et une viscosité comprise entre 38 et 42 mPa.s (4% (w/w) dans l'eau à 20°C).

Dans les compositions selon l'invention, l'agent filmogène est utilisé à des concentrations inférieures ou égales à 20%, de préférence comprises entre 0,5% et 20% en poids par rapport au poids total de la composition et, plus préférentiellement, comprises entre 0.5% et 10%, et de préférence entre 0.5% et 6%, et en particulier 0.5%, 1%, 2%, 3%, 4% et 6%.

La présence d'au moins un agent filmogène permet d'améliorer la tolérance et présente donc un effet particulièrement intéressant dans le cas des formulations comprenant de l'adapalène et du peroxyde de benzoyle. En effet, les dérivés d'acide naphtoïque peuvent être irritants et présenter une action desséchante sur la peau. Il est donc intéressant de diminuer l'irritation induite afin de pouvoir augmenter les doses.

Les compositions de la présente invention peuvent se présenter sous toutes les formes galéniques normalement utilisées pour une application topique, notamment sous forme de dispersions aqueuses, hydroalcooliques ou huileuses, de suspensions, de gels aqueux, anhydres ou lipophiles, d'émulsions (lotions, crèmes ou pommades) de consistance liquide, semi-solide ou solide, obtenues par dispersion d'une phase grasse dans une phase aqueuse (H/E) ou inversement (E/H) en présence ou non d'émulsionnant, ou encore de micro émulsions, de micro capsules, de micro particules ou de dispersions vésiculaires de type ionique et/ou non ionique.

De préférence, les compositions selon l'invention se présentent sous forme d'émulsions (lotions, crèmes, crèmes sans émulsionnant), de suspensions, de gels, et plus préférentiellement sous la forme de gels et d'émulsions.

L'homme du métier veillera à choisir les excipients constituant les compositions selon l'invention en fonction de la forme galénique souhaitée et de manière à ce que les propriétés avantageuses de la composition selon l'invention soient respectées.

La composition de type gel selon l'invention peut en outre notamment comprendre un ou plusieurs des ingrédients suivants :
a) un ou plusieurs agents gélifiants et/ou agents de suspension et/ou agents gélifiants pH-indépendants,
b) optionnellement, un ou plusieurs agents chélatants,
c) optionnellement, un ou plusieurs agents émollients et/ou humectants ;
d) un ou plusieurs agents mouillants,
e) un ou plusieurs additifs.

La composition de type émulsion (crème, lotion, crème sans émulsionnant) selon l'invention peut en outre notamment comprendre un ou plusieurs des ingrédients suivants :
a) un ou plusieurs agents gélifiants et/ou agents de suspension et/ou agents gélifiants pH-indépendants,
b) optionnellement, un ou plusieurs agents chélatants,
c) optionnellement, un ou plusieurs agents émollients et/ou humectants ;
d) un ou plusieurs excipients lipophiles composant la phase grasse ;
e) optionnellement, un ou plusieurs émulsionnants ;
f) un ou plusieurs agents mouillants,
g) un ou plusieurs additifs.

A titre d'exemple non limitatif de gélifiants et/ou agent de suspension et/ou gélifiants pH-indépendants pouvant entrer dans les compositions selon l'invention, on peut citer l'Acrylates/C10-30 Alkyl Acrylate Crosspolymer vendu sous le nom de Pemulen TR-1 ou Pemulen TR-2 par la société Noveon, les carbomers dits non sensibles aux électrolytes, vendus sous le nom d'Ultrez 20®, d'Ultrez 10®, de Carbopol 1382® ou de Carbopol ETD2020NF®, de Carbopol 980®, de Carbopol 981® par la Société Noveon, les polysaccharides avec à titre d'exemples non limitatifs la gomme de xanthane telle que le Xantural180® vendu par la société Kelco, la gellan gum vendu sous le nom de Kelcogel® par la société Kelco, la gomme guar, la cellulose et ses dérivés tel que la microcrystalline cellulose et carboxymethyl cellulose de sodium vendue sous le nom d'Avicel CL-611 par la société FMC Biopolymer, l'hydroxypropylmethylcellulose en particulier le produit vendu sous le nom de Methocel E4M premium par la société Dow Chemical ou l'hydroxyéthylcellulose, en particulier, le produit vendu sous le nom de Natrosol HHX 250® par la société Aqualon, la famille des aluminium magnésium silicates tel que le Veegum K vendu par la société Vanderbilt ,la famille des polymères acryliques couplés à des chaînes hydrophobes tel que le PEG-150/decyl/SMDI copolymer vendu sous le nom de Aculyn 44 (polycondensat comprenant au moins comme éléments, un polyéthylèneglycol à 150 ou 180 moles d'oxyde d'éthylène, de l'alcool décylique et du méthylène bis(4-cyclohexylisocyanate) (SMDI), à 35% en poids dans un mélange de propylèneglycol (39%) et d'eau (26%)), la famille des amidons modifiés tels que l'amidon de pomme de terre modifié vendu sous le nom de Structure Solanace ou bien leurs mélanges et les gélifiants de la famille des polyacrylamides tels que le mélange Sodium acryloyldimethyltaurate copolymer / isohexadecane / polysorbate 80 vendu sous le nom Sepineo P 600 ® (ou de Simulgel 600 PHA®) par la société Seppic, le mélange polyacrylamide / isoparaffine C13-14 / laureth-7 comme, par exemple, celui vendu sous le nom de Sepigel 305 par la société Seppic, la famille des carraghénanes en particulier réparties sous quatre grandes familles : κ, λ, β, w tel que les Viscarin® et les Gelcarin® commercialisés par la société IMCD

Le gélifiant tel que décrit ci-dessus peut être utilisé aux concentrations préférentielles allant de 0,001 à 15 % et, plus préférentiellement, allant de 0,15 à 5%.

A titre de gélifiant préféré, on peut citer la famille des carbomers et en particulier le Carbopol Ultrez-20®, le Carbopol ETD 2020®, la famille des polyacrylamides et en particulier le mélange Sodium acryloyldimethyltaurate copolymer / isohexadecane / polysorbate 80 vendu sous le nom de Sepineo P600® (ou Simulgel 600PHA®), la famille des polysaccharides et en particulier la gomme xanthane vendue sous le nom de Xantural 180®, la famille des celluloses et ses dérivés et en particulier l'hydroxyéthylcellulose vendu sous le nom de Natrosol 250HHX® et l'hydroxypropylmethylcellulose vendu sous le nom de Methocel E4M Prenium®, la famille des polymères acryliques couplés à des chaines hydrophobes et en particulier le PEG-150/decyl/SMDI copolymer vendu sous le nom d'Aculyn 44®.

A titre de gélifiant préféré, on peut citer les carbomers, les polyacrylamides, les polymères acryliques couplés à des chaînes hydrophobes, la cellulose et ses dérivés tel que l'hydroxypropylmethylcellulose ou l'hydroxyéthylcellulose, les polysaccharides et notamment la gomme de xanthane, et en particulier ceux vendus notamment sous les noms Sepineo P 600® (ou de Simulgel 600 PHA®), PEG-150/decyl/SMDI copolymer, de Methocel E4M premium®, Natrosol HHX250®, Xantural180® Carbopol Ultrez 20®.

A titre d'agent de suspension préféré, on peut citer la microcrystalline cellulose et carboxymethyl cellulose de sodium vendue sous le nom d'Avicel CL-611 par la société FMC Biopolymer.

Parmi les agents chélatants, on peut citer à titre d'exemples non limitatifs l'acide éthylène diamine tétra-acétique (EDTA), l'acide diéthylène triamine penta-acétique (DTPA), l'acide éthylène diamine-di (O-hydroxyphényl acétique) (EDDHA), l'acide hydroxy-2-éthylène diamine triacétique (HEDTA), l'acide éthyldiamine-di (O-hydroxy-p-méthyl phényl) acétique (EDDHMA) et l'acide éthylène diamine-di (5-carboxy-2-hydroxyphényl) acétique (EDDCHA).

A titre d'agent chélatant préféré, on peut citer l'acide éthylène diamine tétra-acétique (EDTA) vendu notamment sous le nom Titriplex III®.

Parmi les agents humectants et/ou émollients qui ont pour rôle d'hydrater la peau et de faciliter l'application de la formulation, on utilise optionnellement, sans que cette liste soit limitative, des composés tels que la glycérine et le sorbitol, les sucres (à titre d'exemple le glucose, le lactose), les polyethylene glycol (PEG) (à titre d'exemple le Lutrol E400), l'urée, les acides aminés (à titre d'exemple la sérine, la citrulline, l'arginine, l'asparagine, l'alanine).

A titre d'agent humectant et/ou émollient préféré, on peut citer la glycérine)

Parmi les agents mouillants qui ont pour rôle de diminuer la tension superficielle et de permettre un plus grand étalement du liquide, on utilise préférentiellement, sans que cette liste soit limitative, un agent mouillant pouvant présenter préférentiellement une HLB de 10 à 14, des composés de la famille des Poloxamers et/ou des glycols et plus particulièrement le Synperonic PE/L44 et/ou le Synperonic PE/L62 et/ou des composés tels que le propylène glycol, le dipropylène glycol, le propylène glycol dipélargonate, le lauroglycol, l'éthoxydiglycol, De préférence, les agents mouillants sont sous forme liquide de manière à s'incorporer aisément dans la composition sans qu'il soit nécessaire de la chauffer.

L'agent mouillant particulièrement préféré est le propylène glycol et le Synperonic PE/L44 vendu par la société Uniqema.

La composition selon l'invention peut comprendre un ou plusieurs émulsionnants.

Les émulsionnants sont des composés amphiphiles qui possèdent une partie hydrophobe ayant une affinité pour l'huile et une partie hydrophile ayant une affinité pour l'eau créant ainsi un lien entre les deux phases. Les émulsionnants ioniques ou non ioniques stabilisent donc les émulsions huile/eau en s'adsorbant à l'interface et en formant des couches lamellaires de cristaux liquides.

Le pouvoir émulsifiant des émulsionnants non-ioniques est étroitement lié à la polarité de la molécule. Cette polarité est définie par la HLB (Balance HydrophilelLipophile).

Une HLB élevée indique que la fraction hydrophile est prédominante, et, à l'inverse, une faible HLB indique que la partie lipophile est prédominante. Par exemple, des valeurs de HLB supérieures à environ 10 correspondent à des tensioactifs hydrophiles.

Les émulsionnants peuvent être classés, selon leur structure, sous les termes génériques "ioniques" (anioniques, cationiques, amphotères) ou "non ioniques". Les émulsionnants non ioniques sont des émulsionnants qui ne se dissocient pas en ions dans l'eau et sont donc insensibles aux variations de pH.

On peut citer, à titre d'exemple non limitatif des émulsionnants non ioniques présentant une HLB élevée, les esters de sorbitan tels que le POE(20) sorbitan monooléate, vendu sous le nom de Tween 80 ® (HLB=15) ; le POE(20) sorbitan monostéarate vendu sous le nom de Tween 60 ® (HLB=14.9) ; les éthers d'alcools gras tels que le POE (21) stéaryl ether (HLB= 15.5) vendu sous le nom de Brij 721® par la société Uniqema, ou le ceteareth 20 vendu sous le nom de Eumulgin B2® (HLB de 15,5) par la société Cognis, les esters de polyoxyethylene glycol tel que le glyceryl stéarate and PEG 100 stéarate vendu sous le nom de Arlacel 165 FL ® (HLB=11) par la société Uniqema, le PEG 6 Stéarate et PEG 32 stéarate vendu sous le nom de TEFOSE 1500® (HLB= 10) par la société Gateffossé, les esters de sucre de haut HLB tel que le PEG 20 methyl glucose sesquistéarate vendu sous les noms de glucamate SSE20 (HLB=15) par la société Amerchol et le sucrose laurate vendu sous le nom de Surfhope C-1216® (HLB=16) et le sucrose stéarate vendu sous le nom de Surfhope C-1811® (HLB=11) et de Surhope SE Pharma D-1816®, le sucrose palmitostearate vendu sous le nom de Surfhope SE Pharma D-1616par la société Gattefossé, les esters de polyglycerol. De préférence, lesdits émulsionnants non ioniques de haute HLB, présentent une HLB comprise entre 10 et 18.

On citera, comme exemples non limitatifs les émulsionnants non ioniques de basse HLB (lipophiles), les esters de sorbitan, tels que le monostéarate de sorbitan (HLB = 4.7) vendu sous le nom de Span 60 par la société Uniqema, les esters de glycérol tel que le monostéarate de glycerol vendu sous le nom de Cutina GMSVPH (HLB=3.8) par la sociéta Cognis, les esters de polyethylène glycol tel que le PEG-6 isostéarate vendu sous le nom de Olepal isostéarique® (HLB=8) par la société Gattefossé, les sucroesters de bas HLB tel que le methyl glucose sesquistéarate vendu sous le nom de Glucate SS® (HLB= 6) par la société Amerchol et le sucrose dilaurate vendu sous le nom de Surfhope C-1205 (HLB=5) et le sucrose tristéarate vendu sous le nom de Surfhope C-1803® (HLB=3) par la société Gattefossé.

On peut aussi citer comme autres agents émulsionnants non-ioniques, des cires auto-émulsionnantes qui permettent d'obtenir des émulsions stables facilement par simple dispersion à chaud. A titre d'exemple, le cetearyl alcohol (and) polysorbate 60 vendu sous le nom de Polawax NF par la société Croda, la Polawax GP200 vendu par la société Croda.

De préférence, on utilisera, en tant que système émulsionnant un ou plusieurs couples "émulsionnant non ionique de haute HLB" / "émulsionnant non ionique de faible HLB". Il pourra en particulier s'agir d'un système émulsionnant non ionique comprenant au moins un émulsionnant non ionique présentant une HLB supérieure à environ 10 et au moins un émulsionnant non ionique présentant une HLB inférieure à environ 10.

Le ratio de chacun des deux émulsionnants formantsle couple précité est déterminé le plus souvent par le calcul de la HLB requise de la phase grasse utilisée.

A titre d'émulsionnants préférés on peut citer :
- des émulsionnants hydrophiles de type, Glyceryl Stéarate & PEG-100 Stéarate vendu sous le nom Arlacel 165FL® par la société Uniqema; le PEG 6 stéarate et PEG 32 stéarate vendu sous le nom de TEFOSE 1500® par Gattefossé, le PEG 20 methyl glucose sesquistéarate vendu sous le nom de Glucamate SSE 20® par Amerchol, le sucrose laurate vendu sous le nom de Surfhope SE Pharma D-1216®, le sucrose stéarate vendu sous le nom de Surfhope SE Pharma D-1816®, le sucrose palmitostearate vendu sous le nom de Surfhope SE Pharma D-1616®, le Polyoxyethylene (21) Stéaryl Ether vendu sous le nom Brij721® par Uniqema,et le Cétéareth 20 vendu sous le nom d'Eumulgin B2PH® par Cognis, les esters de sorbitan vendu sous le nom de Tween 80® et Tween 60®.
- des émulsionnants lipophiles de type methyl glucose sesquistéarate tels que le Glucate SS® vendu par Amerchol, le sucrose dilaurate tel que le Surfhope C-1205 et le sucrose tristéarate tel que le Surfhope C-1205.

La composition selon l'invention peut également comprendre une phase grasse. Cette phase grasse peut comprendre par exemple, les huiles végétales, minérales, animales ou synthétiques, des huiles de silicones, et leurs mélanges.

Comme exemple d'huile minérale, on peut citer par exemple des huiles de paraffine de différentes viscosités telles que le Primol 352®, le Marcol 82®, Marcol 152® vendus par la société Esso.

Comme huile végétale, on peut citer l'huile d'amande douce, l'huile de palme, l'huile de soja, l'huile de sésame, l'huile de tournesol, l'huile d'olive.

Comme huile animale ou leur substitut d'origine végétale, on peut citer la lanoline, le squalene, l'huile de poisson, avec comme dérivé le perhydrosqualene vendu sous le nom Sophiderm® par la société Sophim.

Comme huile synthétique, on peut citer un ester tel que le cetearyl isononanoate comme le produit vendu sous le nom de Cetiol SN PH® par la société Cognis France, le diisopropyl adipate comme le produit vendu sous le nom de Crodamol DA® par la société Croda, le palmitate d'isopropyle comme le produit vendu sous le nom de Crodamol IPP® par la société Croda, le caprylique caprique triglycéride tel que Miglyol 812® vendu par la société Univar.

Comme huile de silicone, on peut citer une dimethicone comme le produit vendu sous le nom de Q7-9120 Silicone Fluid® de viscosité de 20 cst à 12500 cst par la société Dow Corning, une cyclomethicone comme le produit vendu sous le nom de ST-Cyclomethicone 5NF® également par la société Dow Corning.

Comme polyisobutene hydrogéné, on peut citer les Parleam® vendus par la société Rossow.

Comme alcool de Guerbet, on peut citer l'octyldodecanol vendu sous le nom d'Eutanol G par la société Cognis.

Pour la composition selon l'invention, les huiles de paraffine et de silicone et plus particulièrement le Marcol 152® et le ST-Cyclomethicone 5NF® sont préférées.

On pourra également mettre des corps gras solides tel que des cires naturelles ou synthétiques, des acides gras tels que l'acide stéarique, des alcools gras tels que le Speziol C18 pharma vendu par la société Cognis, et des agents de texture de type tribehenate, tel que le Compritol 888 vendu par la société Gattefossé ou les huiles de ricin hydrogénées telles que le Cutina HR vendu par la société Cognis. Dans ce cas, l'homme du métier adaptera la température de chauffage de la préparation en fonction de la présence ou non de ces solides.

Les compositions de l'invention peuvent comprendre en outre optionnellement tout additif usuellement utilisé dans le domaine cosmétique ou pharmaceutique tel que des agents neutralisants de type bases ou acides usuels, minéraux ou organiques (à titre d'exemple, la triethanolamine, la solution de soude 10%, le tampon acide citrique/sodium citrate, le tampon acide succinique/succinate de sodium), des filtres solaires, des antioxydants (type Butylhydroxyanisole), des charges, des électrolytes, des conservateurs, des colorants, des parfums, des huiles essentielles, des actifs cosmétiques, des hydratants, des vitamines, des acides gras essentiels, des sphingolipides, des composés autobronzants tels que la DHA, des agents apaisants et protecteurs de la peau tels que l'allantoïne, des agents propénétrants, ou un mélange de ceux-ci et optionnellement, un stabilisant du péroxyde de benzoyle (à titre d'exemple le docusate de sodium, le sodium C14-16 oléfine sulfonate, acide lactique, acide citrique) à une concentration de préférence comprise entre 0% à 2% par rapport au poids total de la composition. Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas, altérées.

Ces additifs peuvent être présents dans la composition à raison de 0% à 20 % en poids par rapport au poids total de la composition.

On peut citer à titre d'exemple de conservateurs le chlorure de benzalkonium, le bronopol, la chlorhexidine, le chlorocrésol et ses dérivés, l'alcool éthylique, le phénoxyéthanol, le sorbate de potassium, la diazolidinylurée, l'alcool benzylique, les parabens, ou leurs mélanges.

A titre d'agent conservateur préféré, on peut citer les parabens, le phénoxyéthanol ou le chlorure de benzalkonium, pris seuls ou en mélange.

Dans un mode préféré, la composition se présente sous forme gel et comprend :
- de 0,1% à 0,3% d'un dérivé de l'acide naphtoïque ;
- de 1 % à 10% de peroxyde de benzoyle
- de 30% à 95% d'eau ;
- de 0.5% à 10% d'agent filmogène;
- de 0,10% à 3% d'un ou plusieurs agents gélifiants et/ou agents de suspension et/ou agents gélifiants pH indépendants ;
- de 0 à 1,5% d'un ou plusieurs agents chélatants
- de 0,1% à 10% d'un ou plusieurs agents mouillants ;
- de 0% à 20% d'un ou plusieurs humectants et/ou agents émollients ;
- de 0 à 20% d'un ou plusieurs additifs.

Dans un mode particulier de réalisation de l'invention, la composition se présente sous forme d'une émulsion huile-dans-eau (H/E) de type lotion, crème, crème sans émulsionnant et comprend :
- de 0,1% à 0,3% d'un dérivé de l'acide naphtoïque ;
- de 1 % à 10% de préférence de 2% à 5% de peroxyde de benzoyle ;
- de 30% à 95% d'eau ;
- de 0.5% à 10% d'agent filmogène ;
- de 0,1% à 3% d'un ou plusieurs agents gélifiants et/ou agents de suspension et/ou agents gélifiants pH indépendants ;
- de 0% à 1,5% d'un ou plusieurs agents chélatants
- de 0,1% à 10% d'un ou plusieurs agents mouillants ;
- de 0% à 20% d'un ou plusieurs humectants et/ou agents émollients ;
- de 0 à 10% d'émulsionnants ;
- de 0,1% à 30% de phase grasse ;
- de 0 à 20% d'un ou plusieurs additifs.

La présente invention a aussi pour objet la composition telle que décrite précédemment à titre de médicament. La composition peut en effet être utilisée comme médicament.

L'invention a également pour objet un procédé de préparation d'une composition telle que décrite précédemment. Un tel procédé est caractérisé en ce qu'il comprend l'étape de mélange d'un véhicule physiologiquement acceptable comprenant au moins un dérivé de l'acide naphtoïque et du peroxyde de benzoyle avec l'agent filmogène, afin d'obtenir une composition dans laquelle ledit dérivé d'acide naphtoïque et du peroxyde de benzoyle est sous une forme dispersée.

L'introduction des autres excipients et additifs éventuels se fera en fonction de la nature chimique des composés et de la forme galénique choisie.

L'introduction de l'agent filmogène est dépendante du polymère. Ainsi, à titre d'exemple, le Mowiol 40-88 est introduit dans la phase aqueuse. En particulier, la présente invention se rapporte à un procédé de préparation d'une composition comprenant les étapes suivantes :
a) préparation de la phase active 1 comprenant l'un des deux actifs
b) préparation de la phase active 2 comprenant l'autre actif
c) préparation de la phase aqueuse
d) (optionnelle) préparation de la phase filmogène
e) mélange des 2 phases actives préparées en a) et b)
f) (optionnelle) préparation d'une phase grasse
g) (optionnelle) émulsification du mélange de phases obtenues en f) et c)
h) mélange de la phase obtenue en g) ou c) avec la phase active unique obtenue en e)
i) (optionnelle) addition du polyacrylamide
j) (optionnelle) Neutralisation de la formule obtenue en h)
k) (optionnelle) addition de la phase filmogène
l) (optionnelle) ajustement en eau

Le procédé principal de préparation de la composition selon l'invention comprend à titre d'exemple les étapes suivantes:

### Etape a: préparation de la phase active 1:

Mélange de l'eau purifiée et du principe actif 1 (adapalène) avec au moins un agent mouillant jusqu'à ce que ledit dérivé d'acide naphtoique soit parfaitement dispersé, afin d'obtenir la phase active 1 ;

### Etape b: préparation de la phase active 2:

Mélange de l'eau purifiée et du principe actif 2 (peroxyde de benzoyle), avec au moins un agent mouillant jusqu'à ce que ledit peroxyde de benzoyle soit parfaitement dispersé, afin d'obtenir la phase active 2 ;

### Etape c : Préparation de la phase aqueuse

Dans un bécher, on introduit sous agitation, si nécessaire à chaud, de l'eau purifiée et, le ou les gélifiants et/ou gélifiants pH indépendants (à l'exception du polyacrylamide) et/ou agents de suspension et optionnellement le ou les agents chélatants, le ou les conservateurs, le ou les émulsionnants hydrophiles, le ou les agents stabilisants, le ou les humectants et/ou émollients, le ou les agents filmogènes.

Etape d : optionnellement, mélange d'au moins un agent filmogène avec de l'eau afin d'obtenir une phase filmogène

### Etape e: le mélange des phases actives

Les deux phases actives obtenues respectivement en a) et b) sont mélangées, l'agitation est maintenue jusqu'à parfaite homogénéisation ;

### Etape f (optionnellement pour l'obtention d'une émulsion): Préparation de la phase grasse :

Mélange, des composés huileux, des corps gras solides et optionnellement des émulsionnants lipophiles, des conservateurs.

Le mélange est chauffé et après homogénéisation, le silicone volatile est introduit en dernier si présent dans la composition.

### Etape g (optionnelle): Emulsification :

A chaud, la phase grasse est introduite dans la phase aqueuse afin de réaliser l'émulsification. Le chauffage est maintenu quelques minutes, puis le produit est refroidi.

### Etape h: addition de la phase active unique

Introduction de la phase active unique obtenue en e) dans la phase aqueuse obtenue en c) pour les gels ou dans la phase obtenue en g) pour les émulsions

### Etape i (optionnelle) : Addition du polyacrylamide

On introduit sous agitation le polyacrylamide à la phase obtenue en h). L'agitation est maintenue jusqu'à parfaite homogénéité.

### Etape j: Neutralisation :

L'agent de neutralisation du gélifiant est introduit si nécessaire, dans la phase obtenue à l'étape h) ou i).

### Etape k: (optionnelle) Addition de la phase filmogène

Addition de la phase filmogène préparée à l'étape d) si le ou les agents filmogènes n'ont pas été introduits dans la phase aqueuse.

### Etape l : (optionnelle) ajustement en eau

Si nécessaire, un ajustement en eau est réalisé.

Le procédé alternatif de préparation de la composition selon l'invention comprend à titre d'exemple les étapes suivantes:
Les principes actifs sont mélangés dans la 1^{ère} étape du procédé décrit ci-dessus ; ainsi les étapes a) et b) sont remplacées par l'étape a') :
   a') préparation de la phase active unique comprenant les deux actifs.

Le procédé est ensuite poursuivi comme décrit à partir de l'étape c) avec suppression de l'étape e).

De manière plus détaillée, le procédé principal de préparation de la composition selon l'invention comprend les étapes suivantes:

### Etape a: préparation de la phase active 1:

Dans un bécher, on introduit sous agitation de l'eau purifiée, le principe actif (Adapalène), les agents mouillants (type Synperonic PE/L62, Synperonic PE/L44, propylène glycol). On laisse sous agitation jusqu'à parfaite dispersion.

### Etape b: préparation de la phase active 2:

Dans un bécher, on introduit sous agitation de l'eau purifiée, le principe actif (peroxyde de benzoyle), les agents mouillants (type Synperonic PE/L62, Synperonic PE/L44, propylène glycol). On laisse sous agitation jusqu'à parfaite dispersion.

### Etape c : Préparation de la phase aqueuse

Dans un bécher, on introduit sous agitation, si nécessaire à chaud, de l'eau purifiée et, le ou les gélifiants (type Carbopol Ultrez 20, ETD2020 NF, Xantural 180, Natrosol 250 HHX) et/ou gélifiants pH indépendants (à l'exception du Simulgel 600PHA) et/ou agents de suspension (type Avicel CL-611) optionnellement le ou les agents chélatants (type EDTA), le ou les conservateurs (type méthyle paraben), le ou les stabilisants (type docusate de sodium), le ou les émollients et/ou humectants (type glycérine), le ou les agents filmogènes (type sodium hyaluronate).

Etape d : optionnellement mélange d'au moins un agent filmogène avec de l'eau afin d'obtenir une phase filmogène ;

### Etape e: le mélange des phases actives

Les deux phases actives obtenues respectivement en a) et b) sont mélangées, l'agitation est maintenue jusqu'à parfaite homogénéisation,

### Etape f (optionnelle): Préparation de la phase grasse :

Mélange, des huiles et corps gras solides (type olépal isostéarique, Cetiol SN PH, Crodamol DA, Speziol C18, Cosbiol) et optionnellement des émulsionnants type (Glucate SS, Glucamate SSE 20, Brij 721, Téfose 1500), des conservateurs (type phénoxyéthanol et propyle paraben).
Le mélange est chauffé et après homogénéisation, le silicone volatile (type Cyclométhicone 5NF) est introduit en dernier si présent dans la composition.

### Etape g (optionnelle): Emulsification :

A chaud, la phase grasse est introduite dans la phase aqueuse afin de réaliser l'émulsification. Le chauffage est maintenu quelques minutes, puis arrêté afin de refroidir la préparation.

### Etape h: addition de la phase active unique

Introduction de la phase active unique obtenue en e) dans la phase aqueuse obtenue en c) pour les gels, et dans la phase obtenue en g) pour les émulsions.

### Etape i (optionnelle) : Addition du Simulgel 600PHA

On introduit sous agitation le Simulgel 600PHA à la phase obtenue en h). Maintenir l'agitation jusqu'à parfaite homogénéité.

### Etape j : Neutralisation :

Si nécessaire, l'agent de neutralisation du gélifiant (type triéthanoloamine ou solution d'hydroxyde de sodium à 10%) est introduit dans la phase obtenue à l'étape h) ou i).

### Etape k : (optionnelle) Addition de la phase filmogène

Addition de la phase filmogène préparée à l'étape d) si le ou les agents filmogènes n'ont pas été introduits dans la phase aqueuse.

### Etape l : (optionnelle) ajustement en eau

Si nécessaire, un ajustement en eau est réalisé.

De manière plus détaillée, le procédé alternatif de préparation de la composition selon l'invention comprend les étapes suivantes: Les principes actifs sont mélangés dans la 1^{ère} étape du procédé décrit ci-dessus ; ainsi les étapes a) et b) sont remplacées par l'étape a') :

### a') Préparation de la phase active unique comprenant les deux actifs

On mélange le dérivé d'acide naphtoïque, le péroxyde de benzoyle avec au moins un agent mouillant, dans de l'eau, jusqu'à ce que ledit peroxyde de benzoyle et ledit dérivé d'acide naphtoïque soient parfaitement dispersés afin d'obtenir une phase active unique selon les mêmes conditions opératoires.

Le procédé est ensuite poursuivi comme décrit à partir de l'étape c) et l'étape e) est supprimée.

L'invention se rapporte à l'utilisation de la nouvelle composition telle que décrite précédemment en cosmétique et en dermatologie.

En particulier, l'invention se rapporte à l'utilisation d'une composition telle que décrite précédemment pour la préparation d'une composition pharmaceutique destinée au traitement et/ou à la prévention des affections dermatologiques liées à un désordre de la kératinisation portant sur la différenciation et sur la prolifération cellulaire notamment pour traiter les acnés vulgaires, comédoniennes, papulopustuleuse, papulocomédoniennes, nodulokystiques, les acnés conglobata, les acnés chéloïdes de la nuque, les acnés miliaires récidivantes, les acnés nécrotiques, les acnés neonatorum, les acnés professionnelles, les acnés séniles, les acnés solaires et les acnés médicamenteuses. Particulièrement, l'invention a pour objet la composition selon l'invention pour son utilisation dans le traitement et/ou la prévention des affections dermatologiques liées à un désordre de la kératinisation portant sur la différenciation et sur la prolifération cellulaire, notamment pour traiter les acnés vulgaires, comédoniennes, papulopustuleuse, papulocomédoniennes, nodulokystiques, les acnés conglobata, les acnés chéloïdes de la nuque, les acnés miliaires récidivantes, les acnés nécrotiques, les acnés neonatorum, les acnés professionnelles, les acnés rosacées, les acnés séniles, les acnés solaires et les acnés médicamenteuses.

Plus particulièrement, l'invention se rapporte à l'utilisation d'une composition telle que décrite précédemment pour la préparation d'une composition pharmaceutique destinée à prévenir et/ou à traiter les acnés vulgaires.

Préférentiellement, lesdites compositions selon l'invention sont administrées par voie topique. Par voie topique, on entend une administration sur la peau, les phanères ou les muqueuses.

En outre, l'invention porte également sur l'utilisation cosmétique d'une composition selon l'invention pour le traitement des peaux à tendance acnéique, pour lutter contre l'aspect gras de la peau ou des cheveux, dans la protection contre les effets néfastes du soleil ou dans le traitement des peaux physiologiquement grasses, ou pour prévenir et/ou lutter contre le vieillissement photo-induit ou chronologique.

La présente invention va maintenant être illustrée au moyen des exemples suivants et des données de stabilités physique et chimique présentées ci-dessous:
La stabilité physique des formulations est contrôlée par une observation macroscopique et microscopique de la formulation conservée à température ambiante à T0, T+1 mois, T+2mois et T+3mois.
A TA, l'observation macroscopique permet de garantir l'intégrité physique des produits.
L'observation microscopique permet d'évaluer la qualité de la dispersion des deux actifs. L'adapalène est observé en lumière fluorescente alors que le peroxyde de benzoyle est observé en lumière polarisée.
On complète la caractérisation du produit fini par une mesure du seuil d'écoulement et de viscosité.
Pour la mesure du seuil d'écoulement, un rhéomètre HAAKE de type VT550 avec un mobile de mesure SVDIN est utilisé.
Les rhéogrammes sont réalisés à 25°C et en vitesse imposée de 0 à 100s⁻¹. Les valeurs de viscosité sont notées aux valeurs de cisaillement constantes de 4 s⁻¹, 20s⁻¹, 100s⁻¹ (γ). Par seuil d'écoulement (τ_{c} exprimé en Pascal) on entend la force nécessaire (contrainte de cisaillement minimum) pour vaincre les forces de cohésion de type Van der Waals et provoquer l'écoulement.
Pour les mesures de viscosité, on utilise des viscosimètres de type Brookfield RVDVII+ et LDVDII+
Les gammes de viscosité mesurables avec ces 2 types de Brookfield sont les suivantes :

   Viscosimètre RVDVII+ : 100 cP - 40 McP

   Viscosimètre LVDVII+ /15 cP - 6 McP

Pour les émulsions, on considère que l'on a à l'instant initial T0 :
- Une crème si la viscosité est supérieure à 30 000 cP
- Une lotion si la viscosité est inférieure à 30 000 cP (Lucinda Bushe, ACPS October 22, 2003 Pharmaceutical nomenclature-Issue and challenges).

La stabilité chimique est assurée par un dosage HPLC de l'Adapalène et par un dosage iodomètrique pour le peroxyde de benzoyle.

Les résultats sont exprimés en g/g d'adapalène et de peroxyde de benzoyle, et en % par rapport au titre théorique.

### EXEMPLES

### Exemple 1: Formulation de type gel contenant de l'adapalène à 0,1% et du peroxyde de benzoyle à 2.5% et le polyvinylalcohol à 2% en tant qu'agent filmogène :

La formule est préparée selon le mode opératoire décrit par le procédé principal et/ou alternatif.

| **Constituants** | **Teneur (% m/m)** |
|---|---|
| Péroxyde de benzoyle | 2,50 |
| Adapalène | 0,10 |
| Propylène glycol | 4,00 |
| Synperonic PE/L44 | 0,20 |
| EDTA | 0.10 |
| Glycérine | 4,00 |
| Docusate de sodium | 0.05 |
| Mowiol 40-88 | 2,00 |
| Simulgel 600PHA | 4.00 |
| Eau purifiée | qsp 100% |

**Données de stabilités :**

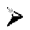 **Stabilité physique :**

| **Caractérisation à T0** | | |
|---|---|---|
| Aspect macroscopique | | Gel blanc |
| Aspect microscopique | | Dispersion des actifs sans agrégats > 100µm |
| **pH** | | 4.91 |
| Données de viscosité | Haake (4s⁻¹/20s⁻¹/100s⁻¹) | 169/254/487 |
| | Brookfield RVDVII+ (S29 ; 5rpm) | 117.10³cP |

| | | |
|---|---|---|
| | | T+1mois |
| Aspect macroscopique | TA | Identique à T0 |
| Aspect microscopique | TA | Identique à T0 |
| pH | TA | 4.37 |
| Rhéologie Haake (4s⁻¹/20s⁻¹/100s⁻¹) | | 186/313/611 |
| Brookfield RVDVII+ (S29 ; 5rpm) | | 114.10³cP |

### ➢ Stabilité chimique

### - Adapalène :

| Conditions de stabilité | Temps | T0 | T+1mois |
|---|---|---|---|
| TA | g/g | 0.098 | 0.097 |
| | % du titre théorique | 98 | 97 |

### - Peroxyde de benzoyle :

| Conditions de stabilité | Temps | T0 | T+1mois |
|---|---|---|---|
| TA | g/g | 2.515 | 2.450 |
| | % du titre théorique | 100.6 | 98 |

## Revendications

1. Composition comprenant, dans un milieu physiologiquement acceptable, au moins un dérivé de l'acide naphtoïque, du péroxyde de benzoyle et au moins un agent filmogène, **caractérisée en ce que** ledit dérivé de l'acide naphtoïque est un composé de formule (I) : 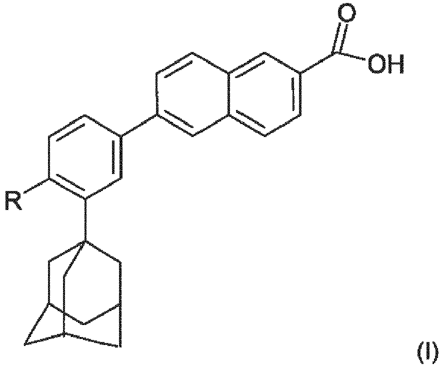 où R représente un atome d'hydrogène, un radical hydroxyle, un radical alkyle ramifié ou non, ayant de 1 à 4 atomes de carbone, un radical alkoxy ayant de 1 à 10 atomes de carbone ou un radical cycloaliphatique, et **en ce que** l'agent filmogène est choisi parmi les alcools polyvinyliques.

2. Composition selon la revendication 1, **caractérisée en ce que** les alcools polyvinyliques présentent un degré de polymérisation compris entre 500 et 5000, un degré d'hydrolyse compris entre 85 et 89%, une viscosité comprise entre 20 et 65 mPa.s (4% (w/w) dans l'eau à 20°C).

3. Composition selon la revendication 1 ou 2, **caractérisée en ce que** l'alcool polyvinylique présente un degré de polymérisation de 4200, un degré d'hydrolyse compris entre 86,7 et 88,7% et une viscosité comprise entre 38 et 42 mPa.s

4. Composition selon l'une des revendications 1 à 3, **caractérisée en ce que** la concentration du dérivé de l'acide naphtoïque est comprise entre 0,001% et 10%, préférentiellement entre 0,01% et 5% et, plus préférentiellement, entre 0,05% et 2% en poids du poids total de la composition, de préférence entre 0,1% et 0,3% en poids du poids total de la composition.

5. Composition selon l'une des revendications précédentes, **caractérisée en ce que** le dérivé d'acide naphtoïque est choisi parmi l'acide 6-[3-(1-adamantyl)-4-methoxyphenyl]-2-naphtoïque, l'acide 6-[3-( 1-adamantyl)-4-hydroxyphenyl]-2-naphtoïque, l'acide 6-[3-(1-adamantyl)-4-decyloxyphenyl]-2-naphtoïque et l'acide 6-[3-(1-adamantyl)-4-hexyloxyphenyl]-2-naphtoïque.

6. Composition selon la revendication 5, **caractérisée en ce que** le dérivé d'acide naphtoïque est l'acide 6-[3-(1-adamantyl)-4-méthoxyphényl]-2-naphtoïque.

7. Composition selon la revendication 6, **caractérisée en ce qu'**elle comprend entre 0,01% et 1% en poids d'acide 6-[3-(1-adamantyl)-4-méthoxyphényl]-2-naphtoïque, préférentiellement entre 0,01% et 0,5%, plus préférentiellement entre 0,1% et 0,4%, par rapport au poids total de la composition.

8. Composition selon la revendication 7, **caractérisée en ce que** la concentration en acide 6-[3-(1-adamantyl)-4-méthoxyphényl]-2-naphtoïque est de 0,1% en poids par rapport au poids total de la composition.

9. Composition selon la revendication 7, **caractérisée en ce que** la concentration en acide 6-[3-(1-adamantyl)-4-méthoxyphényl]-2-naphtoïque est de 0,3% en poids par rapport au poids total de la composition.

10. Composition selon l'une quelconque des revendications précédentes, **caractérisée en ce que** la concentration en agent filmogène est comprise entre 0,5% et 20% en poids par rapport au poids total de la composition, de préférence, entre 0,5% et 10% préférentiellement, entre 0,5% et 6%.

11. Composition selon l'une quelconque des revendications précédentes **caractérisée en ce que** la concentration en peroxyde de benzoyle est comprise entre 1% à 10% en poids par rapport au poids total de la composition, plus particulièrement de 2% à 7%, et préférentiellement de 2,5% à 5%.

12. Composition selon l'une quelconque des revendications précédentes, **caractérisée en ce qu'**elle se présente sous la forme d'une dispersion aqueuse, hydroalcoolique ou huileuse, d'un gel aqueux, anhydre ou lipophile, d'une émulsion de consistance liquide, semi-liquide ou solide obtenue par dispersion d'une phase grasse dans une phase aqueuse ou inversement, ou d'une suspension de consistance molle, semi-liquide ou solide ou encore d'une micro émulsion, de micro capsules, de micro particules ou de dispersions vésiculaires de type ionique et/ou non ionique.

13. Composition selon la revendication 12, **caractérisée en ce qu'**elle se présente sous la forme d'un gel.

14. Composition selon la revendication 12, **caractérisée en ce qu'**elle se présente sous la forme d'une émulsion.

15. Composition selon la revendication 14, **caractérisée en ce que** l'émulsion se présente sous une forme choisie parmi une lotion, une crème, ou une crème sans émulsionnant.

16. Composition selon la revendication 13, **caractérisée en ce qu'**elle comprend dans l'eau :
- de 0,1% à 0,3% d'un dérivé de l'acide naphtoïque ;
- de 1% à 10% de péroxyde de benzoyle ;
- de 30% à 95% d'eau ;
- de 0,5% à 10% d'agent filmogène;
- de 0,10% à 3% d'un ou plusieurs agents gélifiants et/ou agents de suspensions et/ou agents gélifiants pH indépendants ;
- de 0% à 1,5% d'un ou plusieurs agents chélatants ;
- de 0,1% à 10% d'un ou plusieurs agents mouillants ; et
- de 0% à 20% d'un ou plusieurs humectants et/ou agents émollients ;
- de 0 à 20% d'un ou plusieurs additifs.

17. Composition selon l'une des revendications 1 à 12, 14 et 15, **caractérisée en ce qu'**elle comprend dans l'eau:
- de 0,1 % à 0,3% d'un dérivé de l'acide naphtoïque ;
- de 1 à 10% de préférence de 2% à 5% de péroxyde de benzoyle ;
- de 30% à 95% d'eau ;
- de 0,5% à 10% d'agent filmogène;
- de 0,1% à 3% d'un ou plusieurs agents gélifiants et/ou agents de suspensions et/ou agents gélifiants pH indépendants ;
- de 0% à 1,5% d'un ou plusieurs agents chélatants ;
- de 0,1% à 10% d'un ou plusieurs agents mouillants ;
- de 0% à 20% d'un ou plusieurs humectants et/ou agents émollients ;
- de 0 à 10% d'émulsionnants ;
- de 0,1% à 30% de phase grasse ;
- de 0,% à 20% d'un ou plusieurs additifs.

18. Composition selon l'une quelconque des revendications précédentes pour son utilisation à titre de médicament.

19. Procédé de préparation d'une composition selon l'une quelconque des revendications 1 à 18, **caractérisé en ce qu'**il comprend les étapes suivantes :
a) préparation de la phase active 1 comprenant l'un des deux actifs
b) préparation de la phase active 2 comprenant l'autre actif
c) préparation de la phase aqueuse
d) (optionnelle) préparation de la phase filmogène
e) mélange des 2 phases actives préparées en a) et b)
f) (optionnelle) préparation d'une phase grasse
g) (optionnelle) émulsification du mélange de phases obtenues en f) et c)
h) mélange de la phase obtenue en g) ou c) avec la phase active unique obtenue en e)
i) (optionnelle) addition du polyacrylamide
j) (optionnelle) Neutralisation de la formule obtenue en h)
k) (optionnelle) addition de la phase filmogène
l) (optionnelle) ajustement en eau.

20. Procédé de préparation selon la revendication 19, **caractérisé en ce que** les principes actifs sont mélangés dans la 1^{ère} étape du procédé selon la revendication 19; ainsi les étapes a) et b) sont remplacées par l'étape a') :
a') préparation de la phase active unique comprenant les deux actifs ;
le procédé est ensuite poursuivi comme décrit à partir de l'étape c) du procédé selon la revendication 19.

21. Procédé de préparation selon la revendication 19, **caractérisé en ce qu'**il comprend les étapes suivantes:
Etape a: préparation de la phase active 1:
Mélange de l'eau purifiée et du principe actif 1 (adapalène) avec au moins un agent mouillant jusqu'à ce que ledit dérivé d'acide naphtoique soit parfaitement dispersé, afin d'obtenir la phase active 1 ;
Etape b: préparation de la phase active 2:
Mélange de l'eau purifiée et du principe actif 2 (peroxyde de benzoyle), avec au moins un agent mouillant jusqu'à ce que ledit peroxyde de benzoyle soit parfaitement dispersé, afin d'obtenir la phase active 2 ;
Etape c : Préparation de la phase aqueuse
Dans un bécher, on introduit sous agitation, si nécessaire à chaud, de l'eau purifiée et, le ou les gélifiants et/ou gélifiants pH indépendants (à l'exception du polyacrylamide) et/ou agents de suspension et optionnellement le ou les agents chélatants, le ou les conservateurs, le ou les émulsionnants hydrophiles, le ou les agents stabilisants, le ou les humectants et/ou émollients, le ou les agents filmogènes.
Etape d : optionnellement, mélange d'au moins un agent filmogène avec de l'eau afin d'obtenir une phase filmogène ;
Etape e: le mélange des phases actives
Les deux phases actives obtenues respectivement en a) et b) sont mélangées, l'agitation est maintenue jusqu'à parfaite homogénéisation ;
Etape f (optionnellement pour l'obtention d'une émulsion): Préparation de la phase grasse :
Mélange, des composés huileux, des corps gras solides et optionnellement des émulsionnants lipophiles, des conservateurs.
Le mélange est chauffé et après homogénéisation, le silicone volatile est introduit en dernier si présent dans la composition
Etape g (optionnelle): Emulsifcation :
A chaud, la phase grasse est introduite dans la phase aqueuse afin de réaliser l'émulsification. Le chauffage est maintenu quelques minutes, puis le produit est refroidi.
Etape h: addition de la phase active unique
Introduction de la phase active unique obtenue en e) dans la phase aqueuse obtenue en c) pour les gels ou dans la phase obtenue en g) pour les émulsions
Etape i (optionnelle) : Addition du polyacrylamide
On introduit sous agitation le polyacrylamide à la phase obtenue en h). L'agitation est maintenue jusqu'à parfaite homogénéité.
Etape i: Neutralisation :
L'agent de neutralisation du gélifiant est introduit si nécessaire, dans la phase obtenue à l'étape h) ou i).
Etape k: (optionnelle) Addition de la phase filmogène
Addition de la phase filmogène préparée à l'étape d) si le ou les agents filmogènes n'ont pas été introduits dans la phase aqueuse.
Etape l : (optionnelle) ajustement en eau
Si nécessaire, un ajustement en eau est réalisé.

22. Procédé de préparation selon la revendication 21, **caractérisé en ce que** les principes actifs sont mélangés dans la 1^{ère} étape du procédé selon la revendication 21; ainsi les étapes a) et b) sont remplacées par l'étape a') :
a') préparation de la phase active unique comprenant les deux actifs ;
le procédé est ensuite poursuivi comme décrit à partir de l'étape c) du procédé selon la revendication 21.

23. Utilisation d'une composition selon l'une des revendications 1 à 17 pour la préparation d'une composition pharmaceutique destinée au traitement et/ou à la prévention des affections dermatologiques liées à un désordre de la kératinisation portant sur la différenciation et sur la prolifération cellulaire choisies parmi les acnés vulgaires, comédoniennes, papulopustuleuse, papulocomédoniennes, nodulokystiques, les acnés conglobata, les acnés chéloïdes de la nuque, les acnés miliaires récidivantes, les acnés nécrotiques, les acnés neonatorum, les acnés professionnelles, les acnés rosacées, les acnés séniles, les acnés solaires et les acnés médicamenteuses.

24. Utilisation d'une composition selon la revendication 23 pour la préparation d'une composition pharmaceutique destinée à prévenir et/ou à traiter les acnés vulgaires.

## Patentansprüche

1. Zusammensetzung, die, in einem physiologisch annehmbaren Medium, mindestens ein Naphthoesäurederivat, Benzoylperoxid und mindestens eine filmbildende Substanz enthält, **dadurch gekennzeichnet, dass** das Naphthoesäurederivat eine Verbindung mit der Formel (I) ist: 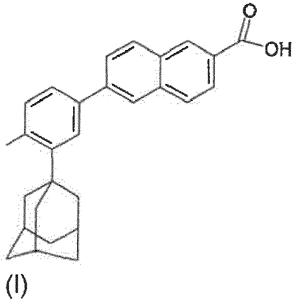 wobei R ein Wasserstoffatom, einen Hydroxylrest, einen verzweigten oder nicht verzweigten Alkylrest mit 1 bis 4 Kohlenstoffatomen, einen Alkoxyrest mit 1 bis 10 Kohlenstoffatomen oder einen cycloaliphatischen Rest darstellt, und dadurch, dass die filmbildende Substanz aus Polyvinylalkoholen ausgewählt ist.

2. Zusammensetzung nach Anspruch 1, **dadurch gekennzeichnet, dass** die Polyvinylalkohole einen Polymerisationsgrad zwischen 500 und 5000, einen Hydrolysegrad zwischen 85 und 89 %, eine Viskosität zwischen 20 und 65 mPa.s (4 % (Gew./Gew.) in Wasser bei 20°C) aufweisen.

3. Zusammensetzung nach Anspruch 1 oder 2, **dadurch gekennzeichnet, dass** der Polyvinylalkohol einen Polymerisationsgrad von 4200, einen Hydrolysegrad zwischen 86,7 und 88,7 % und eine Viskosität zwischen 38 und 42 mPa.s aufweist.

4. Zusammensetzung nach einem der Ansprüche 1 bis 3, **dadurch gekennzeichnet, dass** die Konzentration des Naphthoesäurederivats zwischen 0,001 und 10 Gewichts-%, vorzugsweise zwischen 0,01 und 5 Gewichts-% und bevorzugter zwischen 0,05 und 2 Gewichts-% des Gesamtgewichts der Zusammensetzung liegt, vorzugsweise zwischen 0,1 und 0,3 Gewichts-% des Gesamtgewichts der Zusammensetzung.

5. Zusammensetzung nach einem der vorhergehenden Ansprüche, **dadurch gekennzeichnet, dass** das Naphthoesäurederivat ausgewählt ist aus 6-[3-(1-Adamantyl)-4-methoxyphenyl]-2-Naphthoesäure, 6-[3-(1-Adamantyl)-4-hydroxyphenyl]-2-Naphthoesäure, 6-[3-(1-Adamantyl)-4-decyloxyphenyl]-2-Naphthoesäure und 6-[3-(1-Adamantyl)-4-hexyloxyphenyl]-2-Naphthoesäure.

6. Zusammensetzung nach Anspruch 5, **dadurch gekennzeichnet, dass** das Naphthoesäurederivat 6-[3-(1-Adamantyl)-4-methoxyphenyl]-2-Naphthoesäure ist.

7. Zusammensetzung nach Anspruch 6, **dadurch gekennzeichnet, dass** sie zwischen 0,01 und 1 Gewichts-% 6-[3-(1-Adamantyl)-4-methoxyphenyl]-2-Naphthoesäure, vorzugsweise zwischen 0,01 und 0,5 Gewichts-%, bevorzugter zwischen 0,1 und 0,4 Gewichts-%, bezogen auf das Gesamtgewicht der Zusammensetzung enthält.

8. Zusammensetzung nach Anspruch 7, **dadurch gekennzeichnet, dass** die Konzentration an 6-[3-(1-Adamantyl)-4-methoxyphenyl]-2-Naphthoesäure 0,1 Gewichts-% bezogen auf das Gesamtgewicht der Zusammensetzung beträgt.

9. Zusammensetzung nach Anspruch 7, **dadurch gekennzeichnet, dass** die Konzentration an 6-[3-(1-Adamantyl)-4-methoxyphenyl]-2-Naphthoesäure 0,3 Gewichts-% bezogen auf das Gesamtgewicht der Zusammensetzung beträgt.

10. Zusammensetzung nach einem der vorhergehenden Ansprüche, **dadurch gekennzeichnet, dass** die Konzentration der filmbildenden Substanz zwischen 0,5 und 20 Gewichts-% bezogen auf das Gesamtgewicht der Zusammensetzung, vorzugsweise zwischen 0,5 % und 10 %, bevorzugt zwischen 0,5 % und 6 % beträgt.

11. Zusammensetzung nach einem der vorhergehenden Ansprüche, **dadurch gekennzeichnet, dass** die Konzentration an Benzoylperoxid zwischen 1 und 10 Gewichts-% bezogen auf das Gesamtgewicht der Zusammensetzung, insbesondere von 2 % bis 7 % und bevorzugt von 2,5 % bis 5 % beträgt.

12. Zusammensetzung nach einem der vorhergehenden Ansprüche, **dadurch gekennzeichnet, dass** sie in Form einer wässrigen, wässrig-alkoholischen oder öligen Dispersion, eines wässrigen, wasserfreien oder lipophilen Gels, einer Emulsion mit flüssiger, halbflüssiger oder fester Konsistenz, die durch Dispergieren einer Fettphase in einer wässrigen Phase oder umgekehrt erhalten wird, oder einer Suspension mit weicher, halbflüssiger oder fester Konsistenz oder auch in Form einer Mikroemulsion, von Mikrokapseln, von Mikropartikeln oder Vesikeldispersionen, ionisch und/oder nicht ionisch, vorliegt.

13. Zusammensetzung nach Anspruch 12, **dadurch gekennzeichnet, dass** sie in Form eines Gels vorliegt.

14. Zusammensetzung nach Anspruch 12, **dadurch gekennzeichnet, dass** sie in Form einer Emulsion vorliegt.

15. Zusammensetzung nach Anspruch 14, **dadurch gekennzeichnet, dass** die Emulsion in einer Form ausgewählt aus einer Lotion, einer Creme oder einer Creme ohne Emulgator vorliegt.

16. Zusammensetzung nach Anspruch 13, **dadurch gekennzeichnet, dass** sie in Wasser Folgendes enthält:
- von 0,1 % bis 0,3 % eines Naphthoesäurederivats;
- von 1 % bis 10 % Benzoylperoxid;
- von 30 % bis 95 % Wasser;
- von 0,5 % bis 10 % filmbildende Substanz;
- von 0,10 % bis 3 % von einem oder mehreren Gelbildner(n) und/oder Suspensionsmittel(n) und/oder pH-unabhängigen Gelbildner(n);
- von 0 % bis 1,5 % von einem oder mehreren Chelatbildner(n);
- von 0,1 % bis 10 % von einem oder mehreren Benetzungsmittel(n); und
- von 0 % bis 20 % von einem oder mehreren Feuchthaltemittel(n) und/oder Emollient(s);
- von 0 bis 20 % von einem oder mehreren Zusatzstoffen.

17. Zusammensetzung nach einem der Ansprüche 1 bis 12, 14 und 15, **dadurch gekennzeichnet, dass** sie in Wasser Folgendes enthält:
- von 0,1 % bis 0,3 % eines Naphthoesäurederivats;
- von 1 bis 10 %, vorzugsweise 2 % bis 5 %, Benzoylperoxid;
- von 30 % bis 95 % Wasser;
- von 0,5 % bis 10 % filmbildende Substanz;
- von 0,1 % bis 3 % von einem oder mehreren Gelbildner(n) und/oder Suspensionsmittel(n) und/oder pH-unabhängigen Gelbildner(n);
- von 0 % bis 1,5 % von einem oder mehreren Chelatbildner(n);
- von 0,1 % bis 10 % von einem oder mehreren Benetzungsmittel(n);
- von 0 % bis 20 % von einem oder mehreren Feuchthaltemittel(n) und/oder Emollient(s);
- von 0 bis 10 % Emulgatoren;
- von 0,1 % bis 30 % Fettphase;
- von 0 bis 20 % von einem oder mehreren Zusatzstoffen.

18. Zusammensetzung nach einem der vorhergehenden Ansprüche zur Verwendung als Medikament.

19. Verfahren zur Herstellung einer Zusammensetzung nach einem der Ansprüche 1 bis 18, **dadurch gekennzeichnet, dass** es folgende Schritte umfasst:
a) Herstellen der aktiven Phase 1, die einen der beiden Wirkstoffe enthält,
b) Herstellen der aktiven Phase 2, die den anderen Wirkstoff enthält,
c) Herstellen der wässrigen Phase,
d) (gegebenenfalls) Herstellen der filmbildenden Phase,
e) Vermischen der in a) und b) hergestellten 2 aktiven Phasen,
f) (gegebenenfalls) Herstellen einer Fettphase,
g) (gegebenenfalls) Emulgieren des Gemischs der in f) und c) erhaltenen Phasen,
h) Vermischen der in g) oder c) erhaltenen Phase mit der in e) erhaltenen einzigen aktiven Phase,
i) (gegebenenfalls) Zugeben von Polyacrylamid,
j) (gegebenenfalls) Neutralisieren der in h) erhaltenen Formel,
k) (gegebenenfalls) Zugeben der filmbildenden Phase,
l) (gegebenenfalls) Anpassen in Wasser.

20. Verfahren zur Herstellung nach Anspruch 19, **dadurch gekennzeichnet, dass** die Wirksubstanzen im ersten Schritt des Verfahrens nach Anspruch 19 vermischt werden; Schritt a) und b) werden also durch Schritt a') ersetzt:
a') Herstellen der einzigen aktiven Phase, die die beiden Wirkstoffe enthält;
das Verfahren wird anschließend wie beschrieben ab Schritt c) des Verfahrens nach Anspruch 19 fortgesetzt.

21. Verfahren zur Herstellung nach Anspruch 19, **dadurch gekennzeichnet, dass** es folgende Schritte umfasst:
Schritt a: Herstellen der aktiven Phase 1:
Vermischen von gereinigtem Wasser und der Wirksubstanz 1 (Adapalen) mit mindestens einem Benetzungsmittel, bis das Naphthoesäurederivat vollständig dispergiert ist, damit die aktive Phase 1 erhalten wird;
Schritt b: Herstellen der aktiven Phase 2:
Vermischen von gereinigtem Wasser und der Wirksubstanz 2 (Benzoylperoxid) mit mindestens einem Benetzungsmittel, bis das Benzoylperoxid vollständig dispergiert ist, damit die aktive Phase 2 erhalten wird;
Schritt c:_Herstellen der wässrigen Phase
Unter Rühren werden, bei Bedarf bei erhöhter Temperatur, gereinigtes Wasser und der oder die Gelbildner und/oder pH-unabhängigen Gelbildner (mit Ausnahme von Polyacrylamid) und/oder Suspensionsmittel und gegebenenfalls der oder die Chelatbildner, das oder die Konservierungsmittel, der oder die hydrophilen Emulgatoren, der oder die Stabilisatoren, der oder die Feuchthaltemittel und/oder Emollients, die filmbildende Substanz oder Substanzen in einen Becher gegeben.
Schritt d: gegebenenfalls Vermischen von mindestens einer filmbildenden Substanz mit Wasser zwecks Erhalten einer filmbildenden Phase;
Schritt e: Vermischen der aktiven Phasen
Die beiden aktiven Phasen, die in a) beziehungsweise b) erhalten werden, werden vermischt, es wird bis zur vollständigen Homogenisierung weitergerührt;
Schritt f (gegebenenfalls zum Erhalten einer Emulsion): Herstellen der Festphase :
Vermischen der öligen Bestandteile, der festen Fette und gegebenenfalls der lipophilen Emulgatoren, der Konservierungsmittel.
Das Gemisch wird erwärmt und nach dem Homogenisieren wird das flüchtige Silikon zum Schluss eingebracht, wenn es in der Zusammensetzung vorhanden ist.
Schritt_g (gegebenenfalls) : Emulgieren :
Die Fettphase wird zum Erreichen der Emulsionsbildung bei erhöhten Temperaturen in die wässrige Phase eingebracht. Die Erwärmung wird für einige Minuten aufrechterhalten, anschließend wird das Produkt abgekühlt.
Schritt h: Zugeben der einzigen aktiven Phase Einbringen der in e) erhaltenen einzigen aktiven Phase in die in c) erhaltene wässrige Phase für Gele oder in die in g) erhaltene Phase für Emulsionen,
Schritt i (gegebenenfalls): Zugeben von Polyacrylamid Unter Rühren wird der in h) erhaltenen Phase Polyacrylamid zugegeben. Es wird bis zur vollständigen Homogenität weitergerührt.
Schritt j: Neutralisierung:
Das Mittel zur Neutralisierung des Gelbildners wird bei Bedarf in die in Schritt h) oder i) erhaltene Phase gegeben.
Schritt k: (gegebenenfalls) Zugeben der filmbildenden Phase
Zugeben der in Schritt d) hergestellten filmbildenden Phase, wenn die filmbildende Substanz oder Substanzen nicht in die wässrige Phase gegeben wurden.
Schritt l: (gegebenenfalls) Anpassung in Wasser Bei Bedarf erfolgt eine Anpassung in Wasser.

22. Verfahren zur Herstellung nach Anspruch 21, **dadurch gekennzeichnet, dass** die Wirksubstanzen im ersten Schritt des Verfahrens nach Anspruch 21 vermischt werden; Schritt a) und b) werden also durch Schritt a') ersetzt:
a') Herstellen der einzigen aktiven Phase, die die beiden Wirkstoffe enthält;
das Verfahren wird anschließend wie beschrieben ab Schritt c) des Verfahrens nach Anspruch 21 fortgesetzt.

23. Verwendung einer Zusammensetzung nach einem der Ansprüche 1 bis 17 zur Herstellung einer pharmazeutischen Zusammensetzung, die zur Behandlung und/oder Vorbeugung von Hauterkrankungen bestimmt ist, die mit einer Störung der Verhornung zusammenhängen, die die Zelldifferenzierung und -proliferation betrifft, ausgewählt aus Akne vulgaris, Akne comedonica, Akne papulopustulosa, Akne papulocomedonica, Akne nodulocystica, Akne conglobata, Akne keloidalis nuchae, rezidivierender Akne miliaris, Akne necrotica, Akne neonatorum, Berufsakne, Rosazea, Altersakne, Sonnenakne und Akne medicamentosa.

24. Verwenden einer Zusammensetzung nach Anspruch 23 zur Herstellung einer pharmazeutischen Zusammensetzung zum Vorbeugen und/oder Behandeln von Akne vulgaris.

## Claims

1. Composition comprising, in a physiologically acceptable medium, at least one naphthoic acid derivative, benzoyl peroxide and at least one film-forming agent, **characterized in that** said naphthoic acid derivative is a compound of formula (I): 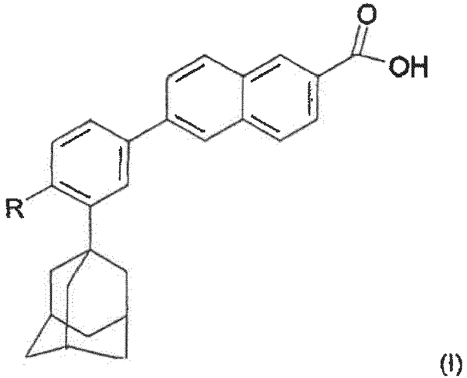 in which R represents a hydrogen atom, a hydroxyl radical, a branched or unbranched alkyl radical containing from 1 to 4 carbon atoms, an alkoxy radical containing from 1 to 10 carbon atoms or a cycloaliphatic radical, and **in that** the film-forming agent is chosen from polyvinyl alcohols.

2. Composition according to Claim 1, **characterized in that** the polyvinyl alcohols have a degree of polymerization between 500 and 5000, a degree of hydrolysis between 85 and 89% and a viscosity between 20 and 65 mPa.s (4% (w/w) in water at 20°C).

3. Composition according to Claim 1 or 2, **characterized in that** the polyvinyl alcohol has a degree of polymerization of 4200, a degree of hydrolysis between 86.7 and 88.7% and a viscosity between 38 and 42 mPa.s.

4. Composition according to one of Claims 1 to 3, **characterized in that** the concentration of the naphthoic acid derivative is between 0.001% and 10%, preferentially between 0.01% and 5%, and more preferentially between 0.05% and 2% by weight of the total weight of the composition, preferably between 0.1% and 0.3% by weight of the total weight of the composition.

5. Composition according to one of the preceding claims, **characterized in that** the naphthoic acid derivative is chosen from 6-[3-(1-adamantyl)-4-methoxyphenyl]-2-naphthoic acid, 6-[3-(1-adamantyl)-4-hydroxyphenyl]-2-naphthoic acid, 6-[3-(1-adamantyl)-4-decyloxyphenyl]-2-naphthoic acid and 6-[3-(1-adamantyl)-4-hexyloxyphenyl]-2-naphthoic acid.

6. Composition according to Claim 5, **characterized in that** the naphthoic acid derivative is 6-[3-(1-adamantyl)-4-methoxyphenyl]-2-naphthoic acid.

7. Composition according to Claim 6, **characterized in that** it comprises between 0.01% and 1% by weight of 6-[3-(1-adamantyl)-4-methoxyphenyl]-2-naphthoic acid, preferentially between 0.01% and 0.5%, more preferentially between 0.1% and 0.4%, relative to the total weight of the composition.

8. Composition according to Claim 7, **characterized in that** the concentration of 6-[3-(1-adamantyl)-4-methoxyphenyl]-2-naphthoic acid is 0.1% by weight relative to the total weight of the composition.

9. Composition according to Claim 7, **characterized in that** the concentration of 6-[3-(1-adamantyl)-4-methoxyphenyl]-2-naphthoic acid is 0.3% by weight relative to the total weight of the composition.

10. Composition according to any one of the preceding claims, **characterized in that** the concentration of film-forming agent is between 0.5% and 20% by weight, relative to the total weight of the composition, preferably between 0.5% and 10%, preferentially between 0.5% and 6%.

11. Composition according to any one of the preceding claims, **characterized in that** the concentration of benzoyl peroxide is between from 1% to 10% by weight relative to the total weight of the composition, more particularly from 2% to 7% and preferentially from 2.5% to 5%.

12. Composition according to any one of the preceding claims, **characterized in that** it is in the form of an aqueous, aqueous-alcoholic or oily dispersion, an aqueous, anhydrous or lipophilic gel, an emulsion of liquid, semi-liquid or solid consistency obtained by dispersing a fatty phase in an aqueous phase or vice versa, or a suspension of soft, semi-liquid or solid consistency, or alternatively a microemulsion, microcapsules, microparticles or vesicular dispersions of ionic and/or nonionic type.

13. Composition according to Claim 12, **characterized in that** it is in the form of a gel.

14. Composition according to Claim 12, **characterized in that** it is in the form of an emulsion.

15. Composition according to Claim 14, **characterized in that** the emulsion is in a form chosen from a lotion, a cream or an emulsifier-free cream.

16. Composition according to Claim 13, **characterized in that** it comprises, in water:
- from 0.1% to 0.3% of a naphthoic acid derivative;
- from 1% to 10% of benzoyl peroxide;
- from 30% to 95% of water;
- from 0.5% to 10% of a film-forming agent;
- from 0.10% to 3% of one or more gelling agents and/or suspending agents and/or pH-independent gelling agents;
- from 0% to 1.5% of one or more chelating agents;
- from 0.1% to 10% of one or more wetting agents; and
- from 0% to 20% of one or more humectants and/or emollients;
- from 0% to 20% of one or more additives.

17. Composition according to one of Claims 1 to 12, 14 and 15, **characterized in that** it comprises, in water:
- from 0.1% to 0.3% of a naphthoic acid derivative;
- from 1 to 10%, preferably from 2% to 5% of benzoyl peroxide;
- from 30% to 95% of water;
- from 0.5% to 10% of a film-forming agent;
- from 0.1% to 3% of one or more gelling agents and/or suspending agents and/or pH-independent gelling agents;
- from 0% to 1.5% of one or more chelating agents;
- from 0.1% to 10% of one or more wetting agents;
- from 0% to 20% of one or more humectants and/or emollients;
- from 0 to 10% of emulsifiers;
- from 0.1% to 30% of fatty phase;
- from 0% to 20% of one or more additives.

18. Composition according to any one of the preceding claims, as a medicament.

19. Process for preparing a composition according to any one of Claims 1 to 18, **characterized in that** it comprises the following steps:
a) preparation of active phase 1 comprising one of the two active agents;
b) preparation of active phase 2 comprising the other active agent;
c) preparation of the aqueous phase;
d) (optional) preparation of the film-forming phase;
e) mixing of the 2 active phases prepared in a) and b);
f) (optional) preparation of a fatty phase;
g) (optional) emulsification of the mixture of phases obtained in f) and c);
h) mixing of the phase obtained in g) or c) with the single active phase obtained in e);
i) (optional) addition of polyacrylamide;
j) (optional) neutralization of the formulation obtained in h);
k) (optional) addition of the film-forming phase;
l) (optional) adjustment with water.

20. Preparation process according to Claim 19, **characterized in that** the active ingredients are mixed in the 1^{st} step of the process according to Claim 19 ; thus, steps a) and b) are replaced with step a') :
a') preparation of the single active phase comprising the two active agents;
the process is then continued as described starting from step c) of the process according to Claim 19.

21. Preparation process according to Claim 19, **characterized in that** it comprises the following steps:
Step a: Preparation of active phase 1:
Mixing purified water and active ingredient 1 (adapalene) with at least one wetting agent until said naphthoic acid derivative is completely dispersed, in order to obtain active phase 1.
Steep b :Preparation of active phase 2 :
Mixing purified water and active ingredient 2 (benzoyl peroxide) with at least one wetting agent until said benzoyl peroxide is completely dispersed, in order to obtain active phase 2.
Step_c: Preparation of the aqueous phase:
Introduced into a beaker, with stirring, if necessary at high temperature, are purified water and the gelling agent(s) and/or pH-independent gelling agent(s) (with the exclusion of polyacrylamide) and/or suspending agent(s) and optionally the chelating agent(s), the preservative(s), the hydrophilic emulsifier(s), the stabilizer(s), the humectant(s) and/or emollient(s), and the film-forming agent(s).
Step d: Optionally, mixing at least one film-forming agent with water in order to obtain a film-forming phase.
Step e:_Mixing_of the active phases:
The two active phases obtained respectively in a) and b) are mixed, and the stirring is maintained until complete homogenization.
Step f (optionally_for obtaining an emulsion): Preparation of the fatty phase:
Mixing of the oily compounds, of the solid fatty substances and optionally of the lipophilic emulsifiers and the preservatives.
The mixture is heated and after homogenization the volatile silicone is introduced last, if present in the composition.
Step g (optional): Emulsification:
At high temperature the fatty phase is introduced into the aqeuous phase in order to carry out the emulsification. The heating is maintained for a few minutes, then the product is cooled.
Step h: Addition of the single active phase:
The single active phase obtained in e) is introduced into the aqueous phase obtained in c) for gels or into the phase obtained in g) for emulsions.
Step i_(optional) : Addition of polyacrylamides:
Polyacrylamide is introduced, with stirring, into the phase obtained in h). The stirring is maintained until complete homogenization.
Step j: Neutralization:
The neutralizing agent for the gelling agent is introduced, if necessary, into the phase obtained in h) or i).
Step k: (optional) Addition of the film-forming phase:
Addition of the film-forming phase prepared in step d) if the film-forming agent(s) have not been introduced into the aqueous phase.
Step l: (optional) Adjustment with water:
If necessary, an adjustment with water is carried out.

22. Preparation process according to Claim 21, **characterized in that** the active ingredients are mixed in the 1^{st} step of the process according to Claim 21; thus, steps a) and b) are replaced with step a'):
a') preparation of the single active phase comprising the two active agents;
the process is then continued as described starting from step c) of the process according to Claim 21.

23. Use of a composition according to one of Claims 1 to 17, for the preparation of a pharmaceutical composition for use in the treatment and/or prevention of dermatological conditions associated with a keratinization disorder related to cell differentiation and proliferation chosen from acne vulgaris, comedonal acne, papulopustular acne, papulocomedonal acne, nodulocystic acne, acne conglobata, acne keloid of the nape of the neck, recurrent miliary acne, acne necrotica, acne neonatorum, occupational acne, acne rosacea, senile acne, solar acne and acne medicamentosa.

24. Use of a composition according to Claim 23, for the preparation of a pharmaceutical composition for use in preventing and/or treating acne vulgaris.
